# EUROPÄISCHE PATENTSCHRIFT

(11) **EP 2 240 207 B1**
(45) Veröffentlichungstag und Bekanntmachung des Hinweises auf die Patenterteilung: **01.05.2013**
(21) Anmeldenummer: 08872022.2
(22) Anmeldetag: 28.11.2008
(51) Int. Cl.: B29C 71/04, G21K 5/10, H01J 33/00, A61L 2/08

(54) **VORRICHTUNG UND VERFAHREN ZUR BEHANDLUNG VON FORMTEILEN MITTELS ENERGIEREICHER ELEKTRONENSTRAHLEN**
DEVICE AND METHOD FOR TREATING FORMED PARTS BY MEANS OF HIGH-ENERGY ELECTRON BEAMS
DISPOSITIF ET PROCÉDÉ POUR TRAITER DES PIÈCES MOULÉES AU MOYEN DE FAISCEAUX ÉLECTRONIQUES HAUTEMENT ÉNERGÉTIQUES

(30) Priorität: 06.02.2008 DE 102008007662
(43) Veröffentlichungstag der Anmeldung: 20.10.2010
(73) Patentinhaber: Robert Bosch GmbH, 70442 Stuttgart (DE)
(72) Erfinder: RAUSCHNABEL, Johannes, 74579 Fichtenau (DE); EBERT, Steffen, 74564 Crailsheim (DE); ULLMANN, Oliver, 74589 Satteldorf (DE); SCHMIEG, Reinhold, 74599 Wallhausen (DE)
(86) Internationale Anmeldenummer: PCT/EP2008/066413
(87) Internationale Veröffentlichungsnummer: WO 2009/097927

(56) Entgegenhaltungen:
- EP-A- 0 340 411
- WO-A-02/39792
- WO-A-2004/110868
- WO-A-2007/107331
- US-A1- 2006 192 140

## Beschreibung

### Stand der Technik

Die Erfindung betrifft eine Vorrichtung und ein Verfahren zur Behandlung von Formteilen mittels energiereicher Elektronenstrahlen, insbesondere auch zum Modifizieren von Stoffeigenschaften an der Oberfläche und im Randbereich dreidimensionaler Formteile nach der Gattung des Vorrichtungs- und des Verfahrensanspruchs.

Es ist bereits aus der WO 2007/107331 A1 bekannt, dass ein dreidimensionales Formteil mittels Elektronen behandelt wird und dabei zwischen zwei Elektronenaustrittsfenstern zu liegen kommt, an denen mindestens ein Elektronenbeschleuniger angeordnet ist. Es sind hier weiterhin Reflektoren derart angeordnet, so dass insbesondere in Seitenbereichen des Formteils, die eventuell zum Beispiel durch Abschattung in nicht genügender Weise mit Elektronen beaufschlagt werden, die Elektronen insbesondere aus den Seitenbereichen auf das Formteil gelenkt werden.

Aus der WO 2004/110868 A1 ist bekannt, dass ein zu behandelndes Objekt in Form einer Folie, eines Gewebes oder einer bahnförmige Ware über Umlenkrollen mit zweimaliger Abwinklung in einen Prozessraum zuführbar ist. Die lichte Öffnung der Elektronenaustrittsfenster kann hierbei auf Millimeterdimensionen beschrankt werden, wie es bereits, ebenso wie die Segmentierung, allgemein aus der Vakuumbeschichtung von Folien bekannt ist.

Aus der DE 40 28 479 C2 ist es weiterhin bekannt, dass zur oberflächennahen Wärmebehandlung von Aluminiumteilen einer Brennkraftmaschine energiereiche Elektronenstrahlen eingesetzt werden. Die Oberfläche wird hierzu in einem flächendeckenden Punkteraster mit dem Elektronenstrahl beaufschlagt, wobei dieser dann von Punkt zu Punkt springt und jeweils eine vorgegebene Zeitspanne bei einem Punkt zum Wärmeeintrag verbleibt.

Es ist somit für sich gesehen bekannt, dass mittels Elektronen eine Energie räumlich und zeitlich determiniert in Materialien eingetragen werden kann, um deren Stoffeigenschaften an der Oberfläche und in vorgegebenen Grenzen auch im Volumen zu verändern. Die dazu benötigten Elektronen werden in der Regel in Elektronenbeschleunigern erzeugt, formiert und beschleunigt, ehe sie bei einer weiteren üblichen Anordnung zum Beispiel über ein meist ebenes Elektronenaustrittsfenster aus dem Hochvakuum an ein höheres Druckniveau im Prozessraum mit dem Formteil geführt werden. Dabei ist meist eine konstante Elektronendichte über der gesamten Ausdehnung des Elektronenaustrittsfensters gewünscht. Nach dem Durchdringen der Gasschicht (zum Beispiel Luft) in der Distanz zwischen Elektronenaustrittsfenster und dem Formteil erreichen die Elektronen die zu behandelnde Produktoberfläche.

Als Elektronenbeschleuniger werden hierzu an sich bekannte Flächenstrahlerzeuger, auch Bandstrahler genannt, oder Axialstrahlerzeuger eingesetzt. Ein üblicher als Axialstrahlerzeuger ausgebildeter Elektronenbeschleuniger umfasst zusätzlich einen Elektronenstrahlablenkraum mit einem Strahlablenksystem, mittels dem ein erzeugter Elektronenstrahl periodisch über das gesamte Elektronenaustrittsfenster und im zeitlichen Mittel in allen Teilbereichen des Fensters mit annähernd gleicher Verweildauer abgelenkt wird.

Dreidimensionale Formteile, wie beispielsweise Verpackungen, medizinische Implantate, OP-Bestecke, Prothesen aus verschiedenen Materialien, zum Beispiel Kunststoff, Papier, Metall oder Keramik, werden in unterschiedlichen Branchen, zum Beispiel in der Verpackungsindustrie, Pharmazie, Medizintechnik oder Kunststoffindustrie eingesetzt. Für bestimmte Anwendungen ist dazu eine Eigenschaftsänderung, zum Beispiel eine Sterilisation, Oberflächenfunktionalisierung, Vernetzung oder Härtung der gesamten Oberfläche und der Randschicht des Formteiles erforderlich.

Es ist weiterhin noch aus der DE 199 42 142 A1 bekannt, Eigenschaften der Oberfläche von Schüttgütern mittels Elektronenenergie zu beeinflussen, indem diese Schüttgüter in mehreren Durchläufen und in veränderter Position an einem Elektronenaustrittsfenster vorbeigeführt werden. Somit sind hieraus, wie aus der WO 2004/110868 A1, gegenüberliegende Emitter aus dem Prinzip der Plasmabehandlung von Folien oder aber von einer Schüttgut (Saatkorn)-Sterilisation bekannt.

Solche Vorrichtungen zum Erzeugen von Elektronen für das Modifizieren von Formteileigenschaften sind derart gestaltet, dass über das gesamte Elektronenaustrittsfenster eine annähernd gleiche Elektronenenergiedichte erzeugt und abgegeben wird. Die bisherigen Transportsysteme führen die Formatteile ohne drehen, bzw. schwenken, immer in derselben Position durch die Behandlungszone der Elektronenbeschleuniger. Damit die gesamte Formteiloberfläche mit Elektronenenergie beaufschlagt wird, wird oft ein Verändern der Position des Formteils während eines Mehrfachdurchlauf vorgenommen. Nachteilig ist hierbei, dass dies mit einem relativ hohen Zeit- und Vorrichtungsaufwand verbunden ist. Das Verändern der Position des Formteils zwischen den einzelnen Durchläufen kann auch nicht wahllos erfolgen, sondern muss derart abgestimmt sein, dass einzelne Oberflächenbereiche in der Summe nicht mit unterschiedlichen Elektronenenergiedichten beaufschlagt werden, was zu unterschiedlichen Eigenschaften führen würde.

Weiterhin ist aus der Druckschrift "Technische Beschreibung "ELECTRON BEAM SURFACE STERILISATION SYSTEM 200 KeV - The Ke VAC S der Firma Linac Technologies" bekannt, dass die gesamte Oberflächenbestrahlung eines dreidimensionalen Formteils während nur eines Durchlaufs mittels Elektronenenergie modifiziert vorgenommen wird, indem mehrere, d.h. mindestens zwei oder drei Elektronenaustrittsfenster derart angeordnet werden, dass diese den Querschnitt des Formteils umschließen, wobei das Formteil zwischen diesen Elektronenaustrittsfenstern hindurchgeführt und somit die gesamte dreidimensionale Oberfläche mit Elektronen beaufschlagt wird.

Es ist an sich auch eine Vorrichtung zum Sterilisieren der Oberfläche von Formteilen mittels Elektronenenergie bekannt, bei der Elektronenbeschleuniger derart angeordnet sind, dass deren zugehörige Elektronenaustrittsfenster ein Volumen mit dem Querschnitt eines gleichschenkligen Dreiecks umschließen, durch das die zu sterilisierenden Formteile in einem Durchlauf hindurchgeführt werden. Mit derartigen Vorrichtungen wird zwar gegenüber den anderen bekannten Lösungen, bei denen ein Formteil in mehreren Durchläufen mit Elektronen beaufschlagt wird, der zeitliche Aufwand verringert, der technische Aufwand ist jedoch wegen des Einsatzes von drei Elektronenbeschleunigern sehr groß.

Es sind weiterhin auch Anordnungen von drei Elektronenaustrittsfenstern bekannt, bei denen die Elektronen jedoch nur mittels eines Elektronenbeschleunigers erzeugt und mit Hilfe eines Ablenksystems auf die drei Elektronenaustrittsfenster aufgeteilt werden.

Alle diese bekannten Lösungen mit mehreren Elektronenaustrittsfenstern nutzen den Vorteil, dass sich die Elektronenbeschleuniger durch deren Dreiecksanordnung nicht oder vernachlässigbar gegenseitig beeinflussen, das heißt, dass die beschleunigten Elektronen eines Elektronenbeschleunigers keine erheblichen Energieanteile auf die jeweils anderen Elektronenbeschleuniger abgeben. Dies ist erforderlich, um den im Elektronenaustrittsfenster absorbierten Energieanteil und damit dessen Betriebstemperatur auf ein unterkritisches Maß zu begrenzen. Bei Überschreitung der Materialeinsatztemperatur würde das empfindliche Material der Fensterabdeckung anderenfalls durch die mechanische Belastung des von außen anliegenden Atmosphärendruckes, im Verhältnis zum Hochvakuum im Inneren des Strahlerzeugers, zerstört. Für die üblicherweise in Elektronenaustrittsfenstern eingesetzte Titanfolie darf eine Maximaltemperatur von etwa 400 °C keinesfalls überschritten werden. Für den Dauerbetrieb geht man von maximal 200-250 °C aus.

Eine aus der US 2,741,704 A bekannte Möglichkeit zur Begrenzung der Temperatur zweier gegenüberliegender Elektronenaustrittsfenster ist die Anordnung eines zusätzlichen Absorbers, wie z. B. eines zumindest teiltransparenten Transportbandes zwischen den Elektronenaustrittsfenstern. Ein erheblicher Energieanteil fällt dann auf den Absorber, was die Einstrahlung zusätzlicher Energie auf das gegenüberliegende Elektronenaustrittsfenster begrenzt.

Bei den bekannten Einrichtungen, bei denen zwei und mehr Elektronenaustrittsfenster ein Formteil umschließen und bei denen über ein gesamtes Elektronenaustrittsfenster eine annähernd gleiche Elektronenenergiedichte abgegeben und ein Formteil nur in einem Durchlauf mit Elektronen beaufschlagt wird, können einzelne Oberflächenteilbereiche des Formteils in Abhängigkeit von dessen Geometrie und dem daraus resultierenden unterschiedlichen Abstand der Oberflächenteilbereiche von einem Elektronenaustrittsfenster mit einer unterschiedlichen Dosis (Energie pro Flächeneinheit oder Energie pro Masseeinheit) an Elektronenenergie beaufschlagt werden.

Um eine bestimmte Eigenschaft an einem Formteil zu realisieren, ist eine bestimmte Dosis an Elektronenenergie erforderlich. Zweckmäßigerweise wird die Leistung der Elektronenerzeuger derart eingestellt, dass an jenen Oberflächenbereichen, an denen die geringste Dosis ankommt, die dort ankommende Dosis genau oder mindestens der Dosis entspricht, die für das Modifizieren der Eigenschaft erforderlich ist. Alle anderen Oberflächenbereiche des Formteils werden zwangsläufig mit einer erhöhten Dosis beaufschlagt. Diese erhöhte Dosis an Energie wird auch als Überdosis bezeichnet. Je höher die Überdosis in einzelnen Bereichen eines Formteils ist, umso stärker weichen die Eigenschaften in diesen Bereichen von den Zielparametern ab. Ein als Überdosisfaktor bezeichneter Parameter gibt an, um welchen Multiplikator eine erforderliche Dosis zum Einstellen einer gewünschten Eigenschaft überschritten wird.

Mit den bekannten Einrichtungen werden somit in Abhängigkeit von der Geometrie der zu behandelnden Formteile in einzelnen Oberflächenbereichen Überdosisfaktoren erreicht, die für viele Anwendungen nicht akzeptabel sind, um hinreichend gleichmäßige Eigenschaften über die gesamte Oberfläche zu realisieren. Für die Erzielung hoher Produktivitäten ist eine angepasst hohe Transportgeschwindigkeit der Formteile erforderlich. Wegen der Proportionalität von Transportgeschwindigkeit und Strahlstrom erfordert die Erzielung einer technologisch vorgegebenen Mindestdosis, die für den Aufgabenbereich der Sterilisation z. B. bei 25 kGy liegt, eine mit der Geschwindigkeit proportionale Erhöhung des Strahlstromes, was zur überproportionalen Erhöhung der Betriebstemperatur der Elektronenaustrittsfenster führt.

### Offenbarung der Erfindung

Die Erfindung geht von einer Vorrichtung zur Behandlung von Formteilen mit energiereichen Elektronenstrahlen aus, bei der die Vorrichtung zwei sich gegenüberliegende stationäre oder bewegliche Elektronenaustrittsfenster aufweist, die einen Prozessraum für das Formteil begrenzen. Erfindungsgemäß ist eine Transporteinrichtung für das Formteil vorhanden, mit der das Formteil an senkrecht zur Transportrichtung im wesentlichen vertikal angeordneten Elektronenaustrittsfenstern vorbei durch den Prozessraum führbar ist, und für den Zutransport des Formteils ist ein gegenüber der Röntgenstrahlung im Prozessraum weitgehend abgeschirmter Kanal angeordnet.

Der Kanal für den Zutransport jeweils vor dem Prozessraum und hinter dem Prozessraum jeweils zweimal derart abgewinkelt, dass der daraus resultierende labyrinthartige Versatz des Kanals die Röntgenstrahlung zum Eingang und zum Ausgang der Vorrichtung hin abschirmt. Im abgewinkelten Kanalabschnitt ist dabei jeweils eine Greifeinrichtung angeordnet, mit der das Formteil aus einem waagerechten Zutransport in eine geneigte, insbesondere auch aufrechte Lage für den Transport im Prozessraum überführbar und anschließend wieder zurückführbar ist.

Diese Einrichtung ist zum Beispiel ein in mehreren Freiheitsgraden steuerbarer Greifer, der vorzugsweise mit einem aufblasbaren und/oder elektromagnetischen Greifelement ausgestattet ist. Durch den vorgeschlagenen Greifermechanismus und das Aufrichten der Formteile entsteht im dazu abgewinkelten Kanal automatisch ein Versatz, der quasi das für die Schirmung erforderliche Labyrinth vorgibt. Die in der Behandlungszone des Prozessraums erzeugte Röntgenstrahlung wird somit zum Eingang und Ausgang abschirmt bzw. reflektiert, wodurch eine sehr kompakte Bauweise bei sehr robuster Mechanik möglich ist und andere sonst notwendige komplizierte Kanal-, Tunnel, oder Schleusenkonstruktionen überflüssig macht.

Im Kanal können dabei in vorteilhafter Weise bleidotierte Glas- oder Kunststofffenster angeordnet sein, durch die mittels einer optischen Sensorik eine Beobachtung und /oder Steuerung der Formteile und deren Transport durchführbar ist. Die optische Sensorik kann dabei aus Lichtschranken und/oder insbesondere elektronischen Kameras bestehen und befindet sich somit in vorteilhafter Weise außerhalb des (Blei-) Kanals.

Vorteilhaft ist es auch, dass bei der erfindungsgemäßen Vorrichtung eine Luftströmung in Richtung des Formteils und an diesem vorbei lenkbar ist. Zur Steuerung der Luftströmung im Prozessraum können vor und/oder hinter dem Formteil klapp- oder sonst wie steuerbare Blenden vorhanden sein, mit denen in vorgegebenen Randbereichen des Formteils die Strömungsquerschnitte zur Steuerung des Drucks im Kanal veränderbar sind.

Die hier vorgeschlagene Luftdruck-Kontrolle im Kanal mit Hilfe von beispielsweise sogenannten "Paddel" als steuerbare Blenden im Prozessraum sind vor allem deshalb vorteilhaft, da die zu behandelnden Formteile kein Schlauch sondern diskrete Gegenstände mit definiertem Abstand zueinander sind und die an den Formteilen entlangströmende Luft also an den Engpässen und Schnittstellen zur Umgebung Schwankungen erlebt, die sich in Druckschwankungen niederschlagen. Um diese Schwankungen zu kompensieren, kann das Paddel so eingesetzt werden, dass immer dann künstlich der Strömungsquerschnitt verringert wird, wenn die Luft zu leicht in angrenzende Bereiche abströmen könnte und damit den Druck absenken würde.

Ein vorteilhafte Ausbildung der erfindungsgemäßen Vorrichtung ergibt sich auch, wenn im Prozessraum in einer, für sich gesehen aus dem eingangs genannten Stand der Technik bekannten Art und Weise mindestens ein Reflektor vorhanden ist, der als weitere seitliche Begrenzung des Prozessraums dient und mit dem Elektronenstrahlen auf die seitlichen Oberflächen und/oder Randschichten des Formteils lenkbar sind. De Elektronenaustrittsfenster und die Transporteinrichtung können auch in einem vorgegebenen Winkel aus der Waagerechten geneigt sein.

Ein vorteilhafte Ausführungsform ergibt sich mit in oder hinter den Elektronenaustrittsfenstern angeordneten Bandstrahlern, die aus nahezu vertikal verlaufenden nebeneinander angeordneten Drähten bestehen, die als Elektronen emittierende Filamente dienen. Alternativ wäre es aber auch möglich, dass anstelle der Bandstrahler eine Scannvorrichtung mit einem Punktstrahler hier angeordnet ist.

Durch die vertikale Position der Elektronenstrahlerzeuger wird bei Bandstrahlern der nicht gänzlich vermeidbare Durchhang des Elektronen emittierenden Filaments, in der Regel ein Wolfram-Draht, für die Emissionscharakteristik neutralisiert. Bei einem von oben nach untern strahlenden Emitter ergibt sich bei einem dann waagerechten Filament, das nur an den Enden gehaltert ist, über die Betriebsdauer ein Durchhang nach unten. Dieser sogenannte Bauch verbiegt den Elektronenvorhang tendenziell zu den Seiten hin, sodass dieser damit nicht auf das Elektronenaustrittsfenster trifft, sondern zu einem relevanten Teil in die Wände und somit in die Anode geht. Bei von unten nach oben strahlenden Emittern, die wegen der Partikelbelastung in der Regel zu vermeiden sind, erfolgt eine Fokussierung des Elektronenvorhangs in einem virtuellen Brennpunkt, und damit erhielten hier die Seiten des zu behandelnden Formteils eine geringere Dosis. Die beiden beschriebenen Bäuche verlangen somit ggf. ein Nachjustieren über die Betriebsdauer, was bei dem erfindungsgemäß vorgeschlagenen Bandstrahler als vertikal gestellter Elektronenstrahlerzeuger nicht notwendig ist, da die Charakteristik des Elektronenvorhangs sich bei optimaler Ausbeute über die Betriebsdauer nicht ändert.

Auch bei den schon bekannten Lösungen mit drei Emittern in Sternanordnung strahlen jeweils zwei Emitter von schräg unten, so dass der Bauch hier den Elektronenvorhang asymmetrisch verschiebt, was auch viel Elektronenenergie in einer Wandseite deponiert, die für die Behandlung des Formteils nicht zur Verfügung steht.

Bei einem Verfahren zur Behandlung von dreidimensionalen Formteilen mit energiereichen Elektronenstrahlen mit einer der zuvor beschriebenen Vorrichtungen wird das Formteil über den Kanal in den Prozessraum geführt und dort vorteilhaft im stationären oder vorbeibewegten Zustand durch einen einmaligen oder mehrfachen Bestrahlungsvorgang mit Elektronen beaufschlagt, wobei der Prozessraum vorteilhaft als Schleuse dient. Der Durchtransport der Formteile erfolgt vorzugsweise im Chargenbetrieb, wobei die Anzahl der Formteile pro Zeiteinheit im Durchtransport unabhängig von der konstanten Durchtransportgeschwindigkeit ist und das abhängig von Taktzeit und Betriebsart ein unterschiedliches Einsetzen der Formteile durch die Greifeinrichtung in das Durchtransportsystem erfolgt.

Nach einer weiteren vorteilhaften Ausführung kann der Prozessraum gegenüber dem Ein- und Auslauf des Kanals jeweils mit Doppelschottschleusen abgetrennt werden, die zum Passieren der zu behandelnden Formteile alternierend geöffnet werden können, wobei auf jeder Seite des Behandlungsbereiches immer ein Schott geschlossen sein muss. In einer bevorzugten Ausführungsform werden die Schotts mit Blei beschichtet, um Röntgenstrahlung aus dem Prozessraum abzuschirmen. In dieser Ausführung ist ein Labyrinth nicht notwendig, da zum Ein- bzw. Auslauf die Starhlung abgeschirmt wird.

Eine weitere vorteilhafte Ausführung beinhaltet eine durch ein Blei-beschichtetes Schott abgetrennte Kammer im Auslaufbereich, die mind. ein Formteil aufnehmen kann, das über ein weiteres Schott oder eine Türe von außen entnommen werden kann. Diese Kammer ist idealerweise senkrecht zum Auslauftransport-Förderband angeordnet und das Formteil kann bei geöffnetem Schott mittels einer translatorischen Bewegung senkrecht zur Auslaufrichtung in die Kammer übergeschoben werden. Nach Schließen des Schotts kann das Formteil dann über die äußere Türe entnommen werden ohne dass der Bestrahlungsbetrieb unterbrochen werden muss. In einer besonders vorteilhaften Ausführung besteht die äußere Türe aus einer Doppeldeckelschleuse, mit der eine sterile Entnahme des Formteils möglich ist.

Eine vorteilhafte Verwendung des obigen Verfahrens oder der obigen Vorrichtung ergibt sich bei einer Behandlung, einer Oberflächenbehandlung mit energiereichen Elektronenstrahlen zum Modifizieren von Kunststoffen, zum Sterilisieren von Produkten/Zwischenprodukten, insbesondere Medizinprodukten, zum Desinfizieren oder/und Sterilisieren von Verpackungen, zum Härten von Beschichtungen oder zum Desinfizieren oder/und Sterilisieren von Gegenständen oder Lebensmitteln.

Der Erfindung löst daher in vorteilhafter Weise eine Reihe technischer Probleme und erlaubt es, eine Vorrichtung mit einer getaktet arbeitenden Transporteinrichtung, und ein Verfahren zu schaffen, mittels denen die Nachteile des Standes der Technik bei der Behandlung mit Elektronenstrahlen überwunden werden. Insbesondere ist die Vorrichtung und das Verfahren geeignet, Eigenschaften dreidimensionaler Formteile mit einem geringen zeitlichen und technischen Aufwand derart zu modifizieren, dass eine hinreichend gleichmäßige Modifizierung der gesamten Oberfläche oder der Randbereiche der Formteile erfolgt und dennoch keine die Produktivität begrenzenden Nachteile aus der Gesamtanordnung der Elektronenbeschleuniger entstehen.

### Kurze Beschreibung der Zeichnung

Die Erfindung wird nachfolgend anhand des in der Figur der Zeichnung gezeigten Ausführungsbeispiels erläutert. Dabei zeigen:

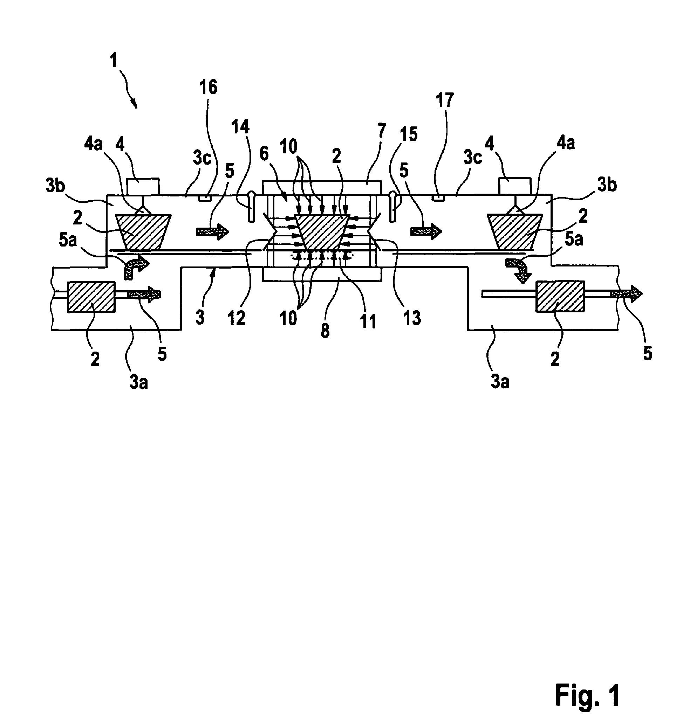

Figur 1 eine schematische Darstellung einer erfindungsgemäßen Vorrichtung zur Behandlung von Formteilen mit Elektronenstrahlen mit einem Prozessraum.

### Ausführungsform der Erfindung

In Figur 1 ist schematisch eine Vorrichtung 1 zur Elektronenbehandlung zum Zwecke der Sterilisation der Oberfläche eines Formteils 2 in einer Draufsicht auf die Transportebene in einem Kanal 3 dargestellt. Das Formteil 2 ist hier dreidimensionaler Gegenstand mit in der Draufsicht rechteckiger Form, wie es aus den Kanalabschnitten 3a am Eingang und am Ausgang ersichtlich ist.

Im Kanalabschnitt 3b ist jeweils ein Greifer 4 angeordnet, der entsprechend gesteuert mit einem Greifelement 4a das Formteil 2 in eine aufrechte Position bringt, so das hier ein trapezförmiger Querschnitt des Formteils 2 in den Kanalabschnitten 3c erkennbar ist. Mit Pfeilen 5 ist die Transportrichtung für die Formteile 2 angedeutet und mit Pfeilen 5a soll die Drehung von der waagerechten in eine aufrechte Position des Formteils 2 symbolisch verdeutlicht werden.

Als Behandlungszone für die Formteile 2 ist hier ein Prozessraum 6 vorhanden, der durch zwei sich parallel gegenüberliegende, sich vertikal in die Zeichenebene hinein erstreckende Elektronenaustrittsfenster 7 und 8 mit jeweils einem hier nicht näher erläuterten, für sich gesehen aus dem Stand der Technik bekannten Bandstrahler zur Erzeugung der energiereichen Elektronenstrahlen 10 begrenzt ist.

Zwischen den beiden Elektronenaustrittsfenstern 7 und 8 wird das Formteil 2 an einem Förderbandsystem 11 kontinuierlich oder mit einem Behandlungsstop durch den Prozessraum hindurchgeführt und dabei wird die gesamte Oberfläche des Formteils 2 mit Elektronenstrahlen 10 beaufschlagt.

An den schrägen Seitenflächen des Formteils 2 in seinem trapezförmigen Querschnitt würde dabei jeweils die geringste Energiedosis an den am weitesten von den Elektronenaustrittsfenstern entfernten Punkten übertragen. Daher sind hier Reflektoren 12 und 13, zum Beispiel aus Gold, als seitliche Begrenzung des Prozessraums 6 derart angeordnet, dass die Elektronenstrahlen vom jeweiligen Elektronenaustrittsfenster 7 oder 8 an die geneigten Flächen der Reflektoren 12 und 13 reflektiert werden. Die ansonsten ungenutzten Randstrahlen der Elektronenstrahlen 10 werden somit durch die Winkelanordnung der Reflektoren 12 und 13 in den Bereich der ohne die Reflektoren niedrigsten Dosis der Elektronenstrahlen 10 auf das Formteil 2 geführt.

Im Inneren des Kanalabschnitts 3c ist hier ein Überdruck notwendig, an den Aus- und Eingängen in den Kanalabschnitten 3a soll jedoch ein Unterdruck herrschen, so dass hier zum Beispiel eine Luftströmung gegen die Transportrichtung zu einer Absaugeinrichtung stattfindet. Daher soll die Luftströmung in Richtung des Formteils 2 und an diesem vorbeigelenkt werden. Zur Steuerung dieser Luftströmung im Prozessraum 6 sind vor und/oder hinter dem Formteil 2 klapp- oder sonst wie steuerbare Blenden 14 und 15 vorhanden, mit denen in vorgegebenen Randbereichen des Formteils 2 die Strömungsquerschnitte zur Steuerung des Drucks im Kanalabschnitt 3c veränderbar sind.

Ferner sind im Kanalabschnitt 3c bleidotierte Glas- oder Kunststofffenster 16 und 17 angeordnet, durch die mittels einer hier nicht gezeigten handelsüblichen optischen Sensorik eine Beobachtung und /oder Steuerung der Formteile 2 und deren Transport durchführbar ist. Die optische Sensorik kann dabei aus außerhalb des Kanalabschnitts 3c oder an anderen Kanalabschnitten angebrachten Lichtschranken und/oder insbesondere elektronischen Kameras bestehen.

Zusammenfassend werden konkrete Ausführungsformen der zuvor als Ausführungsbeispiel erläuterten automatisch, getaktet arbeitenden Vorrichtung zur Bestrahlung des Formteils 2 beschrieben:
- Als Grundbau oder Gehäuse der Vorrichtung 1 bzw. des Kanals 3 kann eine geschweißte Stahlkonstruktion aus Edelstahl in kompakter Labyrinthbauweise mit bleiverstärkter Schutzverkleidung als Röntgenstrahlenschutz verwendet werden, wobei sämtliche Antriebe sowie die Elektronenbeschleuniger der Bandstrahler 7 und 8 außen angeordnet sind und damit optimale Zugänglichkeit ermöglichen. Sämtliche im Inneren verbauten Materialien sollten dabei sowohl Ozon- als auch H₂O₂-beständig ausgelegt sein.
- Ein horizontales Einlaufband kann für die manuelle Aufgabe oder Überleitung des Formteils 2 zum Beispiel von einem sogenannten vorgeschaltetem Bag-Opener und sicherer und präziser Führung des Formteils 2 durch eine Seitenführungen auf einem Band vorgesehen werden.
- Der Greifer 4 als Umsetzstation kann eine entsprechende Handling-Unit mit aufblasbarer Gummi-Dichtung am Greifelement 4a zum formschlüssigen und schonenden Greifen, Anheben, Kippen und linear verschieben zur Übergabe des Formteils 2 an den Durchtransport sein, wobei das Dichtungsmaterial Ozon- und H₂O₂-beständig ist und eventuell eine automatische Drucküberwachung zur Prüfung der Gummi-Dichtung auf Leckagen erfolgt.
- Im Prozessraum 6 kann zum Durchtransport des Formteils 2 ein doppeltes, baulich getrenntes Rundriemensystem als Förderbandsystem 11 zum kontinuierlichen Transport des Formteils 2 durch die Elektronenbeschleuniger-Behandlungszone, bestehend auch aus zwei Edelstahl-Rundriemen zur Führung des Formteils 2 oben und unten, zum Einführen des Formteils 2 in die Elektronenbeschleuniger-Behandlungszone und zwei Edelstahl-Rundriemen, zur Führung des Formteils oben und unten, zum Ausführen des Formteils 2 aus der Elektronenbeschleuniger-Behandlungszone im Prozessraum 6 eingesetzt werden.
- Die Durchtransportebene in der Behandlungszone kann auch im Verhältnis zur Durchtransportrichtung gekippt angeordnet sein, das heißt, die Formteile 2 werden mit einer Neigung von 90° +/- 30° durch die Elektronenbeschleuniger-Behandlungszone im Prozessraum geführt. Hierbei kann dann an der unmittelbaren Übergabestelle zwischen erstem und zweitem Rundriemensystem der Transport berührungslos erfolgen, wodurch sämtliche Oberflächen des Formteils 2 sukzessive der Elektronenstrahlung ausgesetzt werden.
- Der Austransport der Formteile 2 erfolgt analog der Zuführung mit einer Rückdrehung der Formteile 2.
- Für das Bestrahlen eines Formteils 2 innerhalb des Prozessraumes 6 zwischen den beiden Elektronenaustrittsfenstern 7 und 8 stehen verschiedene alternative Möglichkeiten zur Verfügung. So kann ein Formteil 2 mit konstanter Geschwindigkeit durch den Prozessraum 6 geführt und währenddessen mit Elektronenstrahlen 10 beaufschlagt werden. Alternativ besteht auch die Möglichkeit, dass das Formteil 2 in den Prozessraum 6 geführt und dort im stationären Zustand durch einen einmaligen oder mehrfachen Bestrahlungsvorgang mit Elektronen beaufschlagt wird.

## Patentansprüche

1. Vorrichtung (1), zur Behandlung von Formteilen (2) und zur Beaufschlagung der gesamten Oberfläche des Formteils mit Elektronenenergie, mit einer Einrichtung zur Erzeugung von energiereichen Elektronenstrahlen (10), und mit einem Prozessraum (6), welcher durch zwei sich gegenüberliegende stationäre oder bewegliche Elektronenaustrittsfenster (7, 8) begrenzt ist, wodurch die Elektronenstrahlen auf das Formteil (2) leitbar sind, wobei für einen Zutransport des Formteils ein gegenüber einer im Prozessraum (6) entstehbare Röntgenstrahlung weitgehend abgeschirmter Kanal (3, 3a, 3b, 3c) angeordnet ist und der Kanal für den Zutransport jeweils in einem Anschnitt vor dem Prozessraum und hinter dem Prozessraum jeweils zweimal derart abgewinkelt ist, dass der daraus resultierende labyrinthartige Versatz des Kanals die Röntgenstrahlung zum Eingang und zum Ausgang der Vorrichtung hin abschirmt, wobei eine Transporteinrichtung für das Formteil vorhanden ist, mit der das Formteil an den senkrecht zur Transportrichtung im wesentlichen vertikal angeordneten Elektronenaustrittsfenstern vorbei durch den Prozessraum führbar ist und dass im abgewinkelten Kanalabschnitt (3b) jeweils eine Greifeinrichtung angeordnet ist, mit der das Formteil aus einem waagerechten Zutransport in eine geneigte Lage für den Transport im Prozessraum überführbar und anschließend wieder zurückführbar ist.

2. Vorrichtung nach Anspruch 1, derart dass die Greifeinrichtung ein in mehreren Freiheitsgraden steuerbarer Greifer (4) ist, der vorzugsweise mit einem aufblasbaren und/oder elektromagnetischen Greifelement (4a) ausgestattet ist.

3. Vorrichtung nach einem der vorhergehenden Ansprüche, derart, dass im Kanal (3,3a,3b,3c) bleidotierte Glas- oder Kunststofffenster angeordnet sind, durch die mittels einer optischen Sensorik eine Beobachtung und /oder Steuerung der Formteile (2) und deren Transport durchführbar ist

4. Vorrichtung nach Anspruch 3, derart, dass die optische Sensorik aus Lichtschranken und/oder Kameras besteht.

5. Vorrichtung nach einem der vorhergehenden Ansprüche, , dass eine Luftströmung in Richtung des Formteils (2) und an diesem vorbei lenkbar ist.

6. Vorrichtung nach Anspruch 5, derart, dass zur Steuerung der Luftströmung im Prozessraum (6) vor und/oder hinter dem Formteil klapp- oder sonst wie steuerbare Blenden (14,15) vorhanden sind, mit denen in vorgegebenen Randbereichen des Formteils (2) die Strömungsquerschnitte zur Steuerung des Drucks im Kanal (3,3a,3b,3c) veränderbar sind.

7. Vorrichtung nach einem der vorhergehenden Ansprüche, derart, dass im Prozessraum (6) mindestens ein Reflektor (12,13) vorhanden ist, der als weitere seitliche Begrenzung des Prozessraums (6) dient und mit dem die Elektronenstrahlen (10) auf die seitlichen Oberflächen und/oder Randschichten des Formteils (2) lenkbar sind.

8. Vorrichtung nach einem der vorhergehenden Ansprüche, derart, dass hinter den Elektronenaustrittsfenstern (7,8) Bandstrahler angeordnet sind, denen nahezu vertikal angeordnete Drähte als Elektronen emittiebare Filamente dienen.

9. Vorrichtung nach einem der vorhergehenden Ansprüche, derart, dass die Elektronenaustrittsfenstern (7,8) und die Transporteinrichtung in einem Winkel aus der Waagerechten geneigt ist.

10. Vorrichtung nach einem der vorhergehenden Ansprüche, derart, dass im Auslaufbereich eine Kammer senkrecht zum Transport angeordnet ist, die durch ein Schott vom Auslaufkanal getrennt ist und über eine Öffnung in einer Kammerwand zur Entnahme eines Formteils verfügt, die mit einer Türe oder Doppeldeckelschleuse verschlossen ist.

11. Verfahren zur Behandlung von Formteilen mit energiereichen Elektronenstrahlen mit einer Vorrichtung nach einem der vorhergehenden Ansprüche, derart, dass das Formteil (2) über den Kanal (3,3a,3b) in den Prozessraum (6) geführt wird und dort im stationären oder vorbeibewegten Zustand durch einen einmaligen oder mehrfachen Bestrahlungsvorgang mit Elektronenstrahlen (10) beaufschlagt wird, wobei der Prozessraum (6) als Schleuse dient.

12. Verfahren nach Anspruch 11, derart, dass der Durchtransport der Formteile (2) im Chargenbetrieb erfolgt und dass die Anzahl der Formteile (2) pro Zeiteinheit im Durchtransport unabhängig von der konstanten Durchtransportgeschwindigkeit ist und das abhängig von Taktzeit und Betriebsart ein unterschiedliches Einsetzen der Formteile (2) durch die Greifeinrichtung in das Durchtransportsystem erfolgt.

13. Verwendung eines Verfahrens oder einer Vorrichtung nach einem der vorhergehenden Ansprüche, derart, dass die Behandlung, zur Oberflächenbehandlung mit energiereichen Elektronenstrahlen zum Modifizieren von Kunststoffen, zum Sterilisieren von Produkten/Zwischenprodukten, insbesondere Medizinprodukten, zum Desinfizieren oder/und Sterilisieren von Verpackungen, zum Härten von Beschichtungen oder zum Desinfizieren oder/und Sterilisieren von Gegenständen oder Lebensmitteln erfolgt.

## Claims

1. Apparatus (1) for treating formed parts (2) and for subjecting the entire surface of the formed part to electron energy, comprising a device for generating high-energy electron beams (10), and comprising a process chamber (6), which is bounded by two opposing stationary or movable electron discharge windows (7, 8), whereby the electron beams can be directed onto the formed part (2), wherein a channel (3, 3a, 3b, 3c) that is largely shielded from x-radiation that can occur in the process chamber (6) is arranged for delivery of the formed part and the channel for the delivery is angled twice, respectively in a portion upstream of the process chamber and downstream of the process chamber, in each case in such a way that the resultant labyrinthine offset of the channel shields the x-radiation from the inlet and the outlet of the apparatus, wherein there is a transporting device for the formed part, with which the formed part can be guided through the process chamber past the electron discharge windows arranged substantially vertically, perpendicularly in relation to the transporting direction, and that in the angled channel portion (3b) there is respectively arranged a gripping device, with which the formed part can be transferred from a horizontal delivery into an inclined position for the transport in the process chamber and can subsequently be returned again.

2. Apparatus according to Claim 1, such that the gripping device is a grip (4) which can be controlled in multiple degrees of freedom and is preferably equipped with an inflatable and/or electromagnetic gripping element (4a).

3. Apparatus according to one of the preceding claims, such that lead-doped glass or plastic windows, through which observation and/or control of the formed parts (2) and the transporting thereof can be carried out by means of an optical sensor system, are arranged in the channel (3, 3a, 3b, 3c).

4. Apparatus according to Claim 3, such that the optical sensor system consists of light barriers and/or cameras.

5. Apparatus according to one of the preceding claims, such that an air flow can be directed in the direction of the formed part (2) and past it.

6. Apparatus according to Claim 5, such that, for controlling the air flow, in the process chamber (6) there are upstream and/or downstream of the formed part foldable or otherwise controllable baffles (14, 15), with which the flow cross sections are variable in predetermined peripheral regions of the formed part (2) for controlling the pressure in the channel (3, 3a, 3b, 3c).

7. Apparatus according to one of the preceding claims, such that in the process chamber (6) there is at least one reflector (12, 13), which serves as a further lateral boundary of the process chamber (6) and with which the electron beams (10) can be directed onto the lateral surfaces and/or peripheral layers of the formed part (2).

8. Apparatus according to one of the preceding claims, such that arranged downstream of the electron discharge windows (7, 8) are band emitters, for which virtually vertically arranged wires serve as filaments that can emit electrons.

9. Apparatus according to one of the preceding claims, such that the electron discharge windows (7, 8) and the transporting device are inclined at an angle from the horizontal.

10. Apparatus according to one of the preceding claims, such that a chamber is arranged in the outflow region perpendicularly in relation to the transport, is separated from the outflow channel by a bulkhead and has an opening in a chamber wall for the removal of a formed part, which opening is closed by a door or double-lidded lock.

11. Method for treating formed parts with high-energy electron beams by an apparatus according to one of the preceding claims, such that the formed part (2) is guided by the channel (3, 3a, 3b) into the process chamber (6) and is subjected there in a stationary state or a moved-past state to electron beams (10) by a single or repeated irradiation operation, wherein the process chamber (6) serves as a lock.

12. Method according to Claim 11, such that the transporting-through of the formed parts (2) takes place in batch mode and that the number of formed parts (2) transported through per unit of time is independent of the constant transporting-through speed and that, depending on the cycle time and operating mode, a differing insertion of the moulded parts (2) into the transporting-through system is performed by the gripping device.

13. Use of a method or an apparatus according to one of the preceding claims, such that the treatment is performed for surface treatment with high-energy electron beams, for modifying plastics, for sterilizing products/intermediate products, especially medical products, for disinfecting and/or sterilizing packages, for hardening coatings or for disinfecting and/or sterilizing articles or foods.

## Revendications

1. Ensemble (1) destiné à traiter une pièce moulée (2) et appliquer l'énergie d'électrons sur la totalité de la surface de la pièce moulée,
l'ensemble présentant un dispositif de formation de faisceaux (10) d'électrons à haute énergie et un espace de traitement (6) délimité par deux fenêtres (7, 8) de sortie, opposées, stationnaires ou mobiles, par lesquelles les faisceaux d'électrons peuvent être amenés sur la pièce moulée (2),
un canal (3, 3a, 3b, 3c) largement blindé vis-à-vis du rayonnement X qui peut survenir dans l'espace de traitement (6) étant prévu pour apporter la pièce moulée et le canal d'amenée étant coudé deux fois, à savoir une fois dans une partie qui précède l'espace de traitement et une fois dans une partie qui suit l'espace de traitement, de telle sorte que le décalage en labyrinthe ainsi obtenu sur le canal fasse écran au rayonnement X à l'entrée et à la sortie de l'ensemble,
un dispositif de transport de la pièce moulée par lequel la pièce moulée peut être guidée dans l'espace de traitement devant les fenêtres de sortie d'électrons disposées essentiellement à la verticale et perpendiculairement à la direction de transport et
un dispositif de saisie disposé dans chacune des parties coudées du canal (3b) permettant de transférer la pièce moulée depuis une amenée horizontale en position inclinée pour la transporter dans l'espace de traitement, pour ensuite l'y ramener.

2. Ensemble selon la revendication 1, **caractérisé en ce que** le dispositif de saisie est un dispositif de saisie (4) asservi selon plusieurs degrés de liberté et de préférence équipé d'un élément de saisie (4a) gonflable et/ou électromagnétique.

3. Ensemble selon l'une des revendications précédentes, **caractérisé en ce que** le canal (3, 3a, 3b, 3c) présente des fenêtres en verre ou en matière synthétique dopé au plomb qui permettent d'observer à l'aide d'un ensemble de détecteurs optiques et/ou de commander les pièces moulées (2) et leur transport.

4. Ensemble selon la revendication 3, **caractérisé en ce que** l'ensemble de détecteurs optiques est constitué de barrières lumineuses et/ou de caméras.

5. Ensemble selon l'une des revendications précédentes, **caractérisé en ce qu'**un écoulement d'air peut être dirigé vers la pièce moulée (2) et le long de celle-ci.

6. Ensemble selon la revendication 5, **caractérisé en ce que** pour commander l'écoulement d'air, des écrans (14, 15) aptes à être rabattus ou commandés et par lesquels les sections transversales d'écoulement peuvent être modifiées sur des bordures prédéterminées de la pièce moulée (2) pour commander la pression qui règne dans le canal (3, 3a, 3b, 3c) sont prévus en amont et/ou en aval de la pièce moulée dans l'espace de traitement (6).

7. Ensemble selon l'une des revendications précédentes, **caractérisé en ce qu'**au moins un réflecteur (12, 13) qui sert de délimitation latérale supplémentaire de l'espace de traitement (6) et par lequel les faisceaux d'électrons (10) peuvent être orientés sur les surfaces latérales et/ou sur les couches de bordure de la pièce moulée (2) est prévu à l'intérieur de l'espace de traitement (6).

8. Ensemble selon l'une des revendications précédentes, **caractérisé en ce que** des émetteurs en ruban dont les fils disposés sensiblement à la verticale servent de filaments aptes à émettre des électrons sont disposés en aval des fenêtres (7, 8) de sortie d'électrons.

9. Ensemble selon l'une des revendications précédentes, **caractérisé en ce que** les fenêtres (7, 8) de sortie d'électrons et le dispositif de transport sont inclinés par rapport à l'horizontale.

10. Ensemble selon l'une des revendications précédentes, **caractérisé en ce qu'**une chambre séparée du canal de sortie par une cloison étanche et dotée d'une paroi d'enlèvement d'une pièce moulée fermée par une porte ou un sas à double couvercle est disposée perpendiculairement au transport dans la partie de sortie.

11. Procédé de traitement de pièces moulées par des faisceaux d'électrons à haute énergie à l'aide d'un ensemble selon l'une des revendications précédentes, **caractérisé en ce que** la pièce moulée (2) est guidée dans l'espace de traitement (6) par le canal (3, 3a, 3b) pour y être traitée par des faisceaux (10) d'électrons, en position stationnaire ou en déplacement par une opération simple ou multiple d'irradiation, l'espace de traitement (6) servant de sas.

12. Procédé selon la revendication 11, **caractérisé en ce que** le transport des pièces moulées (2) s'effectue par lots et le nombre de pièces moulées (2) transportées par unité de temps est indépendant de la vitesse constante de transport et **en ce qu'**en fonction de la cadence et du mode de fonctionnement, différents nombres de pièces moulées (2) peuvent être insérés dans le système de transport par le dispositif de saisie.

13. Utilisation d'un procédé ou d'un dispositif selon l'une des revendications précédentes, **caractérisée en ce que** le traitement de surface par des faisceaux d'électrons à haute énergie est réalisé pour modifier des matières synthétiques, stériliser des produits ou des produits intermédiaires, en particulier des produits médicaux, pour désinfecter et/ou stériliser les emballages, pour durcir des revêtements, désinfecter et/ou stériliser des objets ou des produits alimentaires.
